# EUROPÄISCHE PATENTSCHRIFT

(11) **EP 2 799 059 B1**
(45) Veröffentlichungstag und Bekanntmachung des Hinweises auf die Patenterteilung: **12.04.2017**
(21) Anmeldenummer: 14001898.7
(22) Anmeldetag: 24.02.2010
(51) Int. Cl.: A61K 8/26, A61K 8/28, A61K 8/39, A61K 8/41, A61Q 15/00, A61K 8/86, A61K 8/37, A61K 8/42, D06F 35/00

(54) **Methode zur Überprüfung der Fleckenbildung durch antitranspirantwirksame Zubereitungen auf Kleidung**
Method for examining the marks formed by anti-perspirant products on clothing
Méthode de vérification de la formation de taches par des préparations anti-transpirantes sur les vêtements

(30) Priorität: 27.02.2009 DE 102009010665
(43) Veröffentlichungstag der Anmeldung: 05.11.2014
(62) Teilanmeldung aus: 10705820.8
(73) Patentinhaber: Beiersdorf AG, 20253 Hamburg (DE)
(72) Erfinder: Biel, Stefan, 21077 Hamburg (DE); Miertsch, Heike, 22459 Hamburg (DE); Urban, Michael, 22523 Hamburg (DE)
(74) Vertreter: Hartmann, Jost

(56) Entgegenhaltungen:
- US-A1- 2006 063 246
- US-A1- 2006 281 652
- NN: "Normen für die Farbmessung", techportal.de , 1. März 2008 (2008-03-01), XP002657625, Gefunden im Internet: URL:http://web.archive.org/web/20080301120 706/http://www.techportal.de/docs/links/fa rbeglanz2003.pdf [gefunden am 2011-08-25]
- EMPA Testmaterials: "Beurteilung von Waschmitteln und Waschverfahren mitkünstlich angeschmutzten Testgeweben", Schweiz , 25. August 2011 (2011-08-25), XP002657626, Gefunden im Internet: URL:http://www.empa-testmaterials.ch/data/ publications/de.evaluationofdetergentsandw ashingprocesseswithartificiallysoiledfabri cs.pdf?PHPSESSID=6d7224a562db3a0e734bd1d3a fb9e27b [gefunden am 2011-08-25]

## Beschreibung

Die Erfindung umfasst eine Methode zur Überprüfung der durch antitranspirantwirksame Substanzen enthaltende kosmetische oder dermatologische Zubereitungen mitverursachten Flecken auf oder in Kleidung.

Bei Anwendung von Antitranspirantien oder Deodorantien reklamieren viele Verbraucher unerwünschte Flecken im Achselbereich in der Kleidung. Es handelt sich dabei häufig um gelbliche Flecken, die auch zu Verkrustungen neigen können. Diese Ablagerungen und Flecken entstehen vor allem durch ein komplexes Zusammenspiel zwischen Produkt, Hautfett, Schweiß und Waschmittel und lassen sich durch herkömmliche Reinigungsverfahren nur schwer entfernen.

Die Flecken können je nach Person unterschiedlich ausgeprägt sein. Eine Ursache sind die in den meisten Deo-AT-Produkten eingesetzten Aluminiumsalze, die als Antitranspirant-wirkstoffe fungieren. Diese hartnäckigen Verfärbungen lassen sich beim Waschen, auch bei Vorbehandlungen mit Fleckenmittel, nicht oder nur schwer vollständig entfernen.

Es gibt zahlreiche Literatur und Patente, die sich mit der Fleckenbildung auf der Haut und der Kleidung und deren Vermeidung bei der Anwendung von Antitranspirantien befassen.

In der EP 1178775 A1 wird die Verwendung von wasserlöslichen Tensiden zur Verbesserung der Abwaschbarkeit der Rückstände von der Haut und Kleidung beschrieben. Es werden Kombinationen von adstringierenden Salzen mit wasserlöslichen, nichtionischen Tensiden beschrieben, die einen raschen Antitranspirantwirkungseintritt und eine hohe Effektivität aufweisen sollen.

Die EP 973492 A1 beschreibt die Verwendung von oberflächenaktiven Substanzen, hauptsächlich nichtionische Emulgatoren, in Antitranspirantien. Die Antitranspirantstiftformulierungen umfassen nichtflüchtige Emollients, ein Träger, z.B. Cyclomethicon, ein Fettalkohol, wie Stearylalkohol, ein Antitransspirantmaterial und ein Tensid. Es wird die Problematik der Bildung von weißen Rückständen auf der Haut und der Kleidung, die sich bei Anwendung von aluminiumhaltigen Antitranspirantien bilden können, beschrieben Hierin wird das Problem des Weißelns der Formulierung durch einen Refraktionsindexabgleich der Bestandteile angegangen.

EP 858317 A1 beschreibt Zubereitungen mit oberflächenaktiven Substanzen mit einem HLB > 10 zur Entfernung der fettigen Rückstände auf der Haut.

EP 696188 A1 beschreibt den Einsatz eines wash-off Agents zur Entfernung der Lipidkomponenten von der Haut, bevorzugt werden dafür Ethoxylate eingesetzt.

Die DE 102008052748, eine frühere Anmeldung der Patentinhaberin, beschreibt, dass in wasserfreien Suspensionen Emulgatoren zur verbesserten Abwaschbarkeit der Formulierung von der Haut eingesetzt werden. Die in der wasserfreien Formel vorteilhaft enthaltenen Strukturgeber können auf der Haut fühlbar wachsige Rückstände hinterlassen. Durch die Anwesenheit von polaren Gruppen an den eingesetzten Emulgatoren wird beim Abwaschen der Formulierung die Affinität zum Wasser erhöht und die Rückstände verschwinden. Dafür eignen sich bevorzugt nichtionische Emulgatoren.

Die Erfindung ist eine Methode zur Überprüfung der durch antitranspirantwirksame Substanzen enthaltende kosmetische oder dermatologische Zubereitungen mitverursachten Flecken auf oder in Kleidung.

Es können ein oder mehrere geladene Tenside in kosmetischen oder dermatologischen Zubereitungen umfassend ein oder mehrere antitanspirantwirksame Stoffe zur Verminderung oder Vermeidung der durch die Zubereitung bedingten Verfleckung auf der Kleidung und zur verbesserten Auswaschbarkeit dieser Flecken verwendet werden. Vornehmlich handelt es sich um Zubereitungen umfassend ein oder mehrere Antitranspirantien, insbesondere solche auf Aluminiumbasis.
Die Zubereitungen umfassend ein oder mehrere geladene Tenside vermindern oder vermeiden die Fleckenbildung in oder auf der Kleidung und verbessern die Auswaschbarkeit von Flecken, die durch die Zubereitung selber mit verursacht wurden, aus der Kleidung

Unter Verminderung bzw. Vermeidung der Fleckenbildung auf Textilien vor und insbesondere nach der Wäsche wird erfindungsgemäß der verringerte b-Wert verstanden, der photometrisch mittels der Farbmaßzahlen im CIE- L*a*b-Farbraum ermittelt wird, und der gegenüber den b-Werten der Textilie, befleckt mit einer Zubereitung mit Antitranspirantmittel aber ohne Tenside, gemessen wird.

Durch ein Paneltest ist gezeigt worden, dass die Fleckenbildung der erfindungsgemäßen Zubereitung in oder auf der Kleidung grundsätzlich vermindert ist bzw. die Kleidung weniger gelblich verfleckt ist. Dies wird nachfolgend erläutert.

Um den Schweißgeruch über einen längeren Zeitraum zu unterdrücken, ist der Einsatz kosmetischer Zubereitungen unerlässlich. Den üblichen kosmetischen Deodorantien liegen unterschiedliche Wirkprinzipien zugrunde, die auch kombiniert werden können: Zum einen werden Deowirkstoffe eingesetzt, die das Wachstum der den Schweißgeruch verursachenden Bakterien unterdrücken. Zu diesen keimhemmenden (bakteriostatischen) Mitteln zählen beispielsweise Triclosan, Chlorhexidin oder die natürlich vorkommenden Verbindungen wie Farnesol und Phenoxyethanol.

Zum anderen werden Antitranspirantien eingesetzt, welche die Schweißabsonderung durch Blockierung der Schweißdrüsenausgänge behindern. In den weitaus meisten Antitranspirantien kann durch Adstringentien - vorwiegend Aluminiumsalze wie Aluminiumhydroxychlorid (Aluminiumchlorohydrat) oder Aluminium/Zirkoniumsalze'- die Bildung des Schweißes reduziert werden.
Auch die Kombination von Adstringentien mit antimikrobiell wirksamen Stoffen in ein- und derselben Zusammensetzung ist gebräuchlich. Ferner werden Parfümstoffe zur Überdeckung des Schweißgeruches eingesetzt.
Bekannt sind weitere antitranspirantwirksame Verbindungen, wie beispielsweise 4-[(2-cyclopentyl-2-hydroxyphenylacetyl)oxy]-1,1-dimethyl-piperidiniumbrömid.

Bekannt und gebräuchlich sind neben den flüssigen Desodorantien wie Zerstäuber und Rollon auch feste Zubereitungen, beispielsweise Deo-Stifte ("Sticks"), Puder, Pudersprays, Intimreinigungsmittel usw.
Als Antitranspirantwirkstoff lassen sich vorteilhaft aktivierte saure Aluminium- und/oder Aluminium/Zirkoniumsalze in wässriger Lösung einarbeiten. Hierbei beziehen sich die beschriebenen Konzentrationsbereiche auf die so genannten Aktivgehalte der Antitranspirant-Komplexe: bei den Aluminium-Verbindungen auf wasserfreie Komplexe, bei den Aluminium/Zirkonium-Verbindungen auf wasser- und pufferfreie Komplexe als Puffer wird hier üblicherweise Glycin verwendet).

Die nachfolgende Auflistung vorteilhaft einzusetzender Antitranspirant-Wirker soll in keiner Weise einschränkend sein:
Aluminium-Salze (der empirischen Summenformel [Al₂(OH)ₘClₙ], wobei m+n=6):
   - Aluminiumchlorhydrat [Al₂(OH)₅Cl] x H₂O
      Standard Al-Komplexe: Locron L, Locron LIC, Locron LIF (Clariant), Chlorhydrol (Reheis), ACH-303 (Summit), Aloxicoll L (Giulini).
      Aktivierte Al-Komplexe: Reach 501 (Reheis), Aloxicoll 51 L
   - Aluminiumsesquichlorhydrat [Al₂(OH)_{4,5}Cl_{15]} x H₂O
      Standard Al-Komplexe: Aloxicoll 31L (Giulini), Westchlor 186 (Westwood Chemicals)
      Aktivierte Al-Komplexe: Reach 301 (Reheis)
   - Aluminiumdichlorhydrat [Al₂(OH)₄Cl₂] x H₂O
Aluminium-Zirkonium-Salze:
   - Aluminium/Zirkonium Trichlorhydrex Glycin [Al₄Zr(OH)₁₃Cl₃] x H₂O x Gly Standard Al/Zr-Komplexe: Rezal 33GC (Reheis), AZG-7164 (Summit)
   - Aluminium/Zirkonium Tetrachlorhydrex Glycin (GLY) [Al₄Zr(OH)₁₂Cl₄] x H₂O x Gly Standard Al/Zr-Komplexe: Rezal 36, Rezal 36G, Rezal 36 GC (Reheis), AZG-368 (Summit), Zirkonal L435G (Giulini), Westchlor ZR 35 BX5, Westchlor ZR 41 (Westwood Chemicals)
   - Aluminium/Zirkonium Pentachlorhydrex Glycin [Al₈Zr(OH)₂₃Cl₅] x H₂O x Gly Standard Al/Zr-Komplexe: Rezal 67 (Reheis), Zirkonal L540, Zirkonal L530 PG (Giulini), Westchlor ZR 80B (Westwood Chemicals)
   - Aluminium/Zirkonium Octachlorhydrex Glycin [Al₈Zr(OH)₂₀Cl₈] x H₂O x Gly:
      Westchlor ZR 82B
   - Reach AZP - 908 SUF activated Aluminum Zirkonium Tetrachlorohydrex Gl
   - Reach AZZ - 902 SUF activated Aluminum Zirkonium Trichlorohydrex Glyc
Ebenso vorteilhaft können aber auch puffer-freie Aluminium/Zirkonium-Salze eingesetzt werden.

Die Antitranspirant-Wirkstoffe aus den zuvor geschilderten Gruppe der adstringierende AT-Mittel, den klassischen AT-Mittel werden in den erfindungsgemäßen Formulierungen in einer Menge von 0,05 bis 40 Gew.%, vorzugsweise von 0,1 bis 20 Gew. %, bezogen auf die Gesamtmasse der Zubereitung, d.h. inklusive ggf. vorhandener Treibgase, eingesetzt.

Nachteilig bei der Verwendung von Antitranspirantien, insbesondere Aluminiumsalzen, ist, wie zuvor ausgeführt, die Bildung von Rückständen in oder auf der Kleidung, die die Kleidung in unschöner Weise verfärben können.

Unter Textilverfleckung werden Verfleckungen, insbesondere im Achselbereich, verstanden. Es handelt sich um Flecken, die nach dem Tragen und/oder Waschen in der Kleidung verbleiben und mit zunehmendem Alter des Kleidungsstückes intensiver werden können. Diese Flecken sind nicht die als "Weißeln" bezeichneten Rückstände auf der Haut oder Kleidung zu verstehen.
Es handelt sich erfindungsgemäß vielmehr um meist gelbliche Verfleckungen, die entstehen, wenn das kosmetische Produkt oder Bestandteile davon beim Schwitzen zusammen mit den Körpersekreten der Achsel auf die Textilie gelangt. Durch das Waschen der Textilie wird ein Teil dieser Ablagerungen ausgewaschen, ein anderer Teil verbleibt als Rückstand auf dem Textil.
Erfindungsgemäß vermag die Zubereitung nicht die Bildung jeglicher Flecken, die sich auf einem Kleidungsstück durch vielfältige Ursachen bilden können, vermindern oder verhindern. Jedoch kann die erfindungsgemäße Zubereitung die Bildung derjenigen Flecken, die durch die Zubereitung selber mit verursacht werden, insbesondere diejenigen durch Antitranspirantstoffe, vermindern oder verhindern sowie deren Auswaschbarkeit verbessern.

Als Maß der Verbesserung bzw. Verminderung wird dabei der Unterschied zur Fleckenbildung bzw. dessen Auswaschung bei der Anwendung der erfindungsgemäßen Zubereitung und der Zubereitung ohne geladene Tenside definiert.
Aber auch grundsätzlich beurteilen unabhängige Beobachter (Panel), dass die Flecken der erfindungsgemäßen Zubereitungen auf der Kleidung gar nicht oder zumindest weniger gelb ausfallen. Eine grundsätzliche Fleckenverminderung ist damit gegeben.

Erfindungsgemäß nicht gemeint sind Verfleckungen, die als weiße Flecken im Falle des direkten Kontaktes eines Deodorants oder Antitranspirants mit dem Stoff entstehen. Dies sind eher die weißliche Ablagerungen der Rezepturbestandteile, z.B. Aluminiumsalze. Diese Flecken sind leicht vermeidbar, wenn dem Produkt vor Anlegen der Kleidung die Möglichkeit zum Trocknen gegeben wird. Diese weißen Rückstände sind in der Regel mechanisch (Bürsten) oder durch Waschen zu entfernen. Diese Problematik des "Weißelns" ist im Stand der Technik ausführlich beschrieben und es werden darin Lösüngsansätze geboten.

Erfindungsgemäß geht es insbesondere um die bekannte gelbliche Verfleckung von Antitranspirantien in oder auf der Kleidung, nachdem das Kleidungsstück gewaschen wurde. Erfindungsgemäß setzt hier auch der Lösungsgedanke der verbesserten Auswaschbarkeit an. Der gelb-Wert des Fleckes wird daher insbesondere über den b-Wert definiert, der photometrisch mittels der Farbmaßzahlen im CIE- L*a*b-Farbraum ermittelt werden kann. Bevorzugt bezieht sich die erfindungsgemäße Verwendung der Fleckenvermeidung bzw. verbesserten Auswaschbarkeit auf Textilien, die Baumwolle enthalten oder aus Baumwolle bestehen.

Erfindungsgemäß werden den kosmetischen Zubereitungen dazu ein oder mehrere geladene Tenside zugesetzt.

Interessanterweise wird durch den Zusatz an geladenen Tensiden, insbesondere an quartären Ammoniumverbindungen, insbesondere an Palmitamidopropyltrimoniumchlorid, zu Zubereitungen, umfassend Antitranspirantien, insbesondere Aluminumverbindungen, die Textilverfleckung signifikant verringert.

Insbesondere Quats mit mindestens einer langen Alkylgruppe haben eine Funktion als waschaktive Substanz und werden z. B. als kationische Tenside in Produkten wie Weichspülern eingesetzt. Sie können auch eine desinfizierende Wirkung haben. Weiterhin werden Quats in Haarpflegemitteln eingesetzt. Sie verbessern die physikalischen Eigenschaften wie Kämmbarkeit, Glanz und antistatisches Verhalten des Haares.
Wie im Stand der Technik zu Varisoft® PATC ausgeführt, verlängern diese Substanzen vermeintlich das Verbleiben von Stoffen, wie Farbe auf den Haaren.

Erfindungsgemäß überraschend hat sich nun herausgestellt, dass die geladenen Tenside, wie quartären Ammoniumverbindung, vornehmlich Palmitamidopropyltrimoniumchloride, die Auswaschbarkeit von Flecken verbessern, was der eigentlichen Eigenschaft der Quats gemäß Produktinformation wiederspricht.

Die Verwendung der geladenen Tenside, insbesondere quartäre Ammoniumverbindungen, in kosmetischen Zubereitungen zur Vermeidung oder Verminderung der Fleckenbildung verursacht durch die kosmetische Zubereitung in oder auf Textilien sowie der verbesserten Auswaschbarkeit dieser Flecken, insbesondere aus Baumwolltextilien, ist damit predestiniert.

Neben der Vermeidung bzw. Verminderung der Fleckenbildung in oder auf der Kleidung ist es überraschenderweise auch gelungen, dass sich die Flecken, die sich nach dem Waschen in der Kleidung gebildet haben, aufgrund des Zusatzes an geladenen Tensiden in der Zubereitung deutlich schwächer erscheinen als ohne den Zusatz an geladenen Tensiden.

Zum Nachweis der verbesserten Auswaschbarkeit und verringerter Fleckenbildung wurden folgende Vergleichsversuche in Form von Probandentests (Panel) durchgeführt.

Ablauf der Untersuchungen:
- Jeder Proband bekommt zwei gleiche Produkte, die sich lediglich im Zusatz des geladenen Tensids (hier: Palmitamidopropyltrimoniumchlorid) unterscheiden. Ein Produkt wird unter der linken, das andere unter der rechten Achsel angewendet.
- Die Produkte werden während des gesamten Testzeitraums angewendet
- Jeder Proband bekommt ein weißes T-Shirt aus Baumwolle.
- Das T-Shirt wird einen Tag getragen und anschließend gewaschen und getrocknet:
   Waschbedingungen: 60°C Haushaltswaschmaschine, Pulverwaschmittel
- Die gewaschenen T-Shirts werden photometrisch auf eine Fleckbildung im Achselbereich untersucht (Ermittlung der Farbmaßzahlen im CIE-L*a*b-Farbraum)
- Die Farbwerte ergeben sich aus der Differenz der Werte im Achselbereich und der Werte eines Referenzareals (unverfleckter Bereich z. B. Schulter oder Rücken)
- Insgesamt wird mindestens 1 Trage- und Waschzyklus durchgeführt, bevorzugt ist die Durchführung von mindestens 3 - 6 Trage- und Waschzyklen
Auswertung der Farbwerte
- dL-Wert: weiß bis grau; ein negativer Wert bedeutet eine Vergrauung des T-Shirts
- da-Wert: rot bis grün; ein negativer Wert bedeutet eine Verstärkung im grünen Bereich
- db-Wert: gelb bis blau; ein positiver Wert bedeutet eine Verstärkung im gelben Bereich

**Zusammensetzung der Testprodukte**

| | Produkt ohne | Produkt mit Palmitamidopropyltrimoniumchlorid |
|---|---|---|
| Aluminiumchlorhydrat | 10,0 | 10,0 |
| Isoceteth-20 | 4,8 | 4,8 |
| Dicaprylylether | 3,0 | 3,0 |
| Glycerylisostearat | 2,4 | 2,4 |
| PEG-150 Distearat | 0,7 | 0,7 |
| Butylenglykol | 3,0 | 3,0 |
| Varisoft ® PATC | | 3,0 |
| Parfüm | 1,0 | 1,0 |
| Wasser | 75,1 | 72,1 |
| Farbwerte nach 6 Zyklen | | |
| dL | -2,4 | -1,3 |
| da | -0,6 | -0,2 |
| db | 3,6 | 2,1 |

Die Vergleichszubereitung ohne geladenes Tensid führte zu einer geringeren Auswaschbarkeit, d.h. die Flecken waren im Vergleich zum erfindungsgemäßen Produkt grauer (Differenz L-Wert -1,1), grüner (Differenz α-Wert -0,4) und vor allem gelber (Differenz b-Wert 1,5).

Die Farbwerte wurden aus allen Tests mit allen (7) Probanden zusammengeführt und gemittelt.

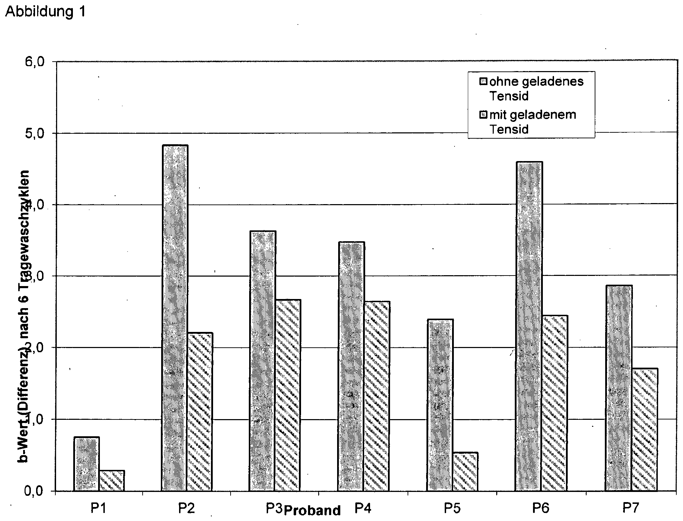

Des Weiteren wurden die Flecken visuell beurteilt. Auf einer Skala von 0 bis 5 haben die Probanden die Fleckbildung beurteilt, wie stark sich ein Fleck im Achselbereich ausgebildet hat. Ein Wert von 0 bedeutet keinen Fleck, ein Wert von 5 bedeutet ein starker Fleck. Im Mittelwert wurde die Fleckintensität für ein Produkt ohne geladenes Tensid mit 3,7 und die mit geladenem Tensid mit 2,7 bewertet
In der Abbildung 1 ist am Beispiel des b-Wertes (gelb) der Unterschied für die Formulierungen mit und ohne geladenes Tensid für 7 einzelne Probanden nach 6 Trage- und Waschzyklen dargestellt. Bei allen Probanden führt das erfindungsgemäße Produkt zu einer geringeren Gelbfärbung.

Als Beleg der verbesserten Auswaschbarkeit und verminderten Fleckenbildung der erfindungsgemäßen Zubereitungen wurden weitere in-vitro und in-vivo Untersuchungen durchgeführt.

### In vitro Methode zur Bestimmung der Fleckintensität

Materialien:
Textil: weiß, 100% Baumwolle (Jersey-Qualität)
   Testsubstanzen/Medien
   a.) Sebum: Sebum nach BEY
   b.) Schweiß: Humanschweiß (künstl. Achselschweiß)
   c.) Vergleichsprodukte
   Das Auftragen des Schweißes ist optional.
Wasserqualität: Leitungswasser (vorzugsweise mittelhart bis hart)
Waschmittel: handelsübliche Waschmittel, z. B. Spee Megaperls
Waschmaschine: Linitest+ von ATLAS
Das Sebum bewirkt, dass der Fleck einen vergleichbaren Gelbanteil bekommt. Ohne Sebum werden die Flecken eher grau. Eine derartige Methode ist in der Literatur bereits beschrieben: The Trouble with Stains. SPC July page 25-28; Materialien wie Sebum nach BEY sind beispielsweise als Standard-Testschmutz bei der WfK-Testgewebe GmbH erhältlich.

### Auftragsmengen:

a.) Zubereitung: 1 g auf einem Areal von 15 cm x 2,5 cm der Textilie, entspricht 27 mg/cm²
b.) Humanschweiß: 0,5 g auf einem Areal von 15 cm x 2,5 cm, entspricht 13 mg/cm²
c.) Sebum: 0,25 g auf einem Areal von 15 cm x 2,5 cm, entspricht 7 mg/cm²

In weiteren Vergleichsuntersuchungen können die Auftragsmengen in folgenden Bereichen gewählt werden, um eine höheren Praxisbezug zu gewährleisten, was an den gezeigten Ergebnissen jedoch keine signifikanten Änderungen ergibt.
Produkt: möglicher Bereich: 10 mg/cm² bis 50 mg/cm², vorzugsweise 13 mg/cm² bis 40 mg/cm²
Sebum: möglicher Bereich: 2 mg/cm² bis 15 mg/cm², vorzugsweise 5 mg/cm² bis 10 mg/cm²
Humanschweiß: möglicher Bereich: 5 mg/cm² bis 40 mg/cm², vorzugsweise 7 mg/cm² bis 30 mg/cm²
Verhältnis Produkt zu Sebum 1:1 bis 7:1, vorzugsweise 2:1 bis 5:1
Verhältnis Produkt zu Humanschweiß 1:3 bis 7:1, vorzugsweise 1:1 bis 4:1

### Farbmessung:

photometrisch, Ermittlung der Farbmaßzahlen im CIE- L*a*b-Farbraum Differenzbildung gegenüber einem unverfleckten Referenzmaterial Mittelwert aus mind. 5 Messungen pro Areal Zur Auswertung wird vorzugsweise der b-Wert verwendet (Gelbwert)

### Ablauf:

1. Vorwaschen und Trocknen des Textilzuschnitte
2. Kennzeichnung der Areale
3. Bevorzugtes Auftragen der Medien in folgender Reihenfolge: a.) Zubereitung, b.) ggf. Schweiß, c.) Sebum, nach jedem Material mind. 10 min warten, bis es eingezogen ist Alternativ können die Substanzen vollständig oder teilweise vorab miteinander vermischt werden und dann in dieser Mischung aufgetragen werden.
4. Einlagern der Proben bei 38°C und 80% Luftfeuchte vorzugsweise für mind. 12 Stunden
5. Waschen der Textilproben einzeln im Linitest (z. B. 1g pulverförmiges Waschmittel auf 300 ml Leitungswasser, Waschtemperatur 60°C) und Auspülen mit kaltem Leitungswasser
6. Trocknen bei Raumtemperatur
7. Farbmessung auf den verfleckten Arealen und einem unverfleckten Referenzareal
8. Wiederholung des Ablaufes von Punkt 2 bis 7 mindestens 1 mal, vorzugsweise 4 mal

Die Methode zur Überprüfung der durch antitranspirantwirksame Substanzen enthaltende kosmetische oder dermatologische Zubereitungen mitverursachten Flecken auf oder in Kleidung ist demnach erfindungsgemäß. Es werden dabei nacheinander
a.) Zubereitung, in einer Menge von 10 mg/cm² bis 50 mg/cm²,
b.) ggf. Humanschweiß, in einer Menge von 5 mg/cm² bis 40 mg/cm² und
c.) Sebum, in einer Menge von 2 mg/cm² bis 15 mg/cm²,
auf die gleiche Stelle der Kleidung aufgetragen. Anschließend wird
- ggf. die Kleidung bei 38°C und 80% Luftfeuchte vorzugsweise für mind. 12 Stunden eingelagert,
- die Kleidung einzeln gewaschen,
- ggf. mit kaltem Leitungswasser ausgespült,
- bei Raumtemperatur getrocknet und
photometrische die verfleckten Areale gegen unverfleckte Referenzareale der gleichen Kleidung mittels Farbmaßzahlen im CIE- L*a*b-Farbraum vermessen.

### In vivo Methode zur Bestimmuna der Fleckintensität (wie zuvor beschrieben)

### Materialien:

Textil: weißes, 100% Baumwolle, T-Shirt
Waschmittel: handelsübliche Waschmittel, Persil
Waschmaschine: handelsübliches Gerät
Wasserqualität: mittelhartes bis hartes Wasser

### Farbmessung:

photometrisch, Ermittlung der Farbmaßzahlen im CIE- L*a*b-Farbraum Differenzbildung gegenüber einem unverfleckten Referenzmaterial z. B. im Schulterbereich des T-Shirts Mittelwert aus mind. 5 Messungen pro Areal Zur Auswertung wird vorzugsweise der b-Wert verwendet (Gelbwert)

### Ablauf:

1. Vorwaschen der T-Shirts
2. Verteilung des Produktes / der Produkte (möglich ist jedes Produkt nacheinander oder vorzugsweise ein Direktvergleich zweier Produkte rechte Achsel vs. Linke Achsel)
3. Produktanwendung mind. 3 Tage zur Vorkondionierung
4. Tragen des T-Shirts nach Produktanwendung morgens für mind. 8 Stunden
5. Waschen des T-Shirts bei vorzugsweise 60°C (abhängig vom Textilmaterial)
6. Trocknen in einem handelsüblichen Wäschetrockner
7. Farbmessung auf den verfleckten Arealen im Achselbereich und einem unverfleckten Referenzareal
8. Wiederholung des Ablaufes von Punkt 4 bis 7 mindestens 3 mal

**Ergebnisse der in vitro Versuche (nach 4 Zyklen)**

| **Produkte als Roller / Gew.%** | **Vgl. 1** | **P2** | **P3** |
|---|---|---|---|
| Aluminum Chlorhydrate | 10,0 | 10,0 | 10,0 |
| Isoceteth-20 | 4,8 | 4,8 | 4,8 |
| Dicaprylylether | 3,0 | 3,0 | 3,0 |
| Glycerylisostearat | 2,4 | 2,4 | 2,4 |
| PEG-150 Distearat | 0,7 | 0,7 | 0,7 |
| Butylenglykol. | 3,0 | 3,0 | 3,0 |
| Varisoft ® PATC | | 3,0 | 3,0 |
| Plantapon LC7 | | | 1,0 |
| Wasser | 76,1 | 73,1 | 72,1 |
| *b-Wert* | *8,1* | *5,1* | *3,6* |

| | | | |
|---|---|---|---|
| Varisoft® PATC: 60% Palmitamidopropyltrimonium Chloride + 40% Propylene Glycol | | | |

| **Produkte als Roller /Gew.%** | **Vgl 2** | **P4** | **P5** |
|---|---|---|---|
| Aluminum Chlorhydrate | 10,0 | 10,0 | 10,0 |
| Isoceteth-20 | 4,8 | 4,8 | 4,8 |
| Paraffinum Liquidum | 3,0 | 3,0 | 3,0 |
| Glycerylisostearat | 2,4 | 2,4 | 2,4 |
| Butylenglykol | 3,0 | 3,0 | 3,0 |
| PEG-150 Distearat | 0,7 | 0,7 | 0,7 |
| Varisoft® PATC | - | 1,0 | 2,0 |
| Plantapon LC7 | - | 1,0 | 1,0 |
| Parfüm | 1,0 | 1,0 | 1,0 |
| Wasser | ad 100 | ad 100 | ad 100 |
| *b-Wert* | *3,9* | *2,4* | *1,9* |

| **Produkte als Stifte / Gew.%** | **Vgl 3** | **P6** |
|---|---|---|
| Aluminum Zirconium Tetrachlorohydrex GLY | 16,0 | 16,0 |
| Glycerylstearat | 0,6 | 0,6 |
| Stearylalkohol | 20,0 | 20,0 |
| Hydrogenated Castor Oil | 1,5 | 1,5 |
| PPG-14 Butylether | 15,0 | 15,0 |
| Cyclomethicone | 41,9 | 38,9 |
| Talkum | 4,0 | 4,0 |
| Varisoft® PATC | - | 3,0 |
| Parfüm | 1,0 | 1,0 |
| *b-Wert* | *7,2* | *4,9* |

| **Produkte als Stifte /Gew.%** | **Vgl 4** | **P7** | **P8** | **P9** |
|---|---|---|---|---|
| Aluminum Zirconium Tetrachlorohydrex GLY | 16 | 16 | 16 | 16 |
| Glycerylstearat | 2 | 2 | 2 | 2 |
| Stearylalkohol | 20 | 20 | 20 | 20 |
| Hydrogenated Castor Oil | 1,5 | 1,5 | 1,5 | 1,5 |
| Caprylic/Capric Triglyceride | 10 | 10 | 10 | 10 |
| Paraffinum Liquidum | 12 | 12 | 12 | 12 |
| PPG-14 Butylether | 20 | 19 | 18 | 18 |
| Cyclomethicone | 13,5 | 13,5 | 13,5 | 13,5 |
| Talkum | 4 | 4 | 4 | 4 |
| Varisoft® PATC | - | 1 | 1 | 1,5 |
| Plantapon LC 7 | - | | 1 | 0,5 |
| Parfüm | 1 | 1 | 1 | 1 |
| *b-Wert* | *5,6* | *4,3* | *3,4* | *3,6* |

| **Produkte als Aerosole /Gew.%** | **Vgl 5** | **P10** |
|---|---|---|
| Aluminum Chlorohydrate | 20 | 20 |
| Cetyl PEG/PPG-10/1 Dimethicone | 1,5 | 3,0 |
| Polyglyceryl-2 Dipolyhydroxystearate | 1,5 | - |
| Cyclomethicone | 11,75 | 11,75 |
| C12-15 Alkylbenzoate | 8 | 8 |
| Dicaprylyl Ether | 6 | 6 |
| Octyldodecanol | 8 | 8 |
| Parfüm | 3,5 | 3,5 |
| Varisoft® PATC | - | 3,0 |
| Wasser | ad 100 | ad 100 |
| *b-Wert* | *7,2* | *4,1* |

| | | |
|---|---|---|
| Aerosol Gew.% bezogen auf Emulsion, Abfüllung 30 Gew.% Emulsion : 70 Gew.% Treibgas (Gemisch aus Propan, Isobutan, Butan) | | |

**Ergebnisse der in vivo Versuche:**

| 10 Zyklen, 26 Probanden | | |
|---|---|---|
| | Vgl 6 | P11 |
| Aluminumchlorhydrat | 10,0 | 10,0 |
| Isoceteth-20 | 4,8 | 4,8 |
| Dicaprylylether | 3,0 | 3,0 |
| Glycerylisostearat | 2,4 | 2,4 |
| PEG-150 Distearat | 0,7 | 0,7 |
| Butylenglykol | 3,0 | 3,0 |
| Varisoft® PATC | | 3,0 |
| Plantapon LC7 | | 1,0 |
| Parfüm | 1,1 | 1,1 |
| Wasser | 75,0 | 71,0 |
| *b-Wert* | *0,55* | *0,38* |

Die Vergleichsuntersuchungen zeigen, dass die erfindungsgemäßen Zubereitungen P1 - P11, umfassend mindestens ein geladenes Tensid, eine verbesserte Auswaschbarkeit und verminderte Fleckenbildung gegenüber Zubereitungen ohne diese geladenen Tenside (Vgl 1 - Vgl 6) aufweisen.

## Patentansprüche

1. Methode zur Überprüfung der durch antitranspirantwirksame Substanzen enthaltende kosmetische oder dermatologische Zubereitungen mitverursachten Flecken auf oder in Kleidung indem
a.) Zubereitung, in einer Menge von 10 mg/cm² bis 50 mg/cm²,
b.) ggf. Humanschweiß, in einer Menge von 5 mg/cm² bis 40 mg/cm² und
c.) Sebum, in einer Menge von 2 mg/cm² bis 15 mg/cm²,
auf die gleiche Stelle der Kleidung aufgetragen werden,
anschließend
• ggf. Einlagern der Kleidung bei 38°C und 80% Luftfeuchte vorzugsweise für mind. 12 Stunden,
• Waschen der Kleidung einzeln,
• ggf. Auspülen mit kaltem Leitungswasser,
• Trocknen bei Raumtemperatur und
• photometrische Vermessung der verfleckten Areale gegen unverfleckte Referenzareale der gleichen Kleidung mittels Farbmaßzahlen im CIE- L*a*b-Farbraum.

## Claims

1. Method for examining stains on or in clothing, caused by cosmetic or dermatological preparations containing antiperspirant substances, by applying
a.) a preparation in an amount of 10 mg/cm² to 50 mg / cm²,
b.) optionally human perspiration in an amount of 5 mg / cm² to 40 mg / cm² and
c.) sebum in an amount of 2 mg/cm² to 15 mg/cm² onto the same patch of the clothing,
subsequently
• optionally storing the clothing at 38°C and 80% humidity preferably for at least 12 hours,
• washing the clothing singly,
• optionally rinsing with cold tap water,
• drying at room temperature and
• photometrically measuring the stained areas versus unstained reference areas of the same clothing by means of colourimetric values in the CIE L*a*b colour space.

## Revendications

1. Procédé pour tester les taches provoquées par des préparations cosmétiques ou dermatologiques contenant des substances à activité antitranspirante sur ou dans des vêtements, dans lequel on applique
a) la préparation en une quantité de 10 mg/cm² à 50 mg/cm²,
b) le cas échéant de la transpiration humaine en une quantité de 5 mg/cm² à 40 mg/cm²,
c) du sébum, en une quantité de 2 mg/cm² à 15 mg/cm², au même endroit des vêtements, ensuite
- on stocke le cas échéant les vêtements à 38°C et à une humidité de l'air de 80% pendant au moins 12 heures,
- on lave les vêtements séparément,
- on rince le cas échéant avec de l'eau de ville froide,
- on sèche à température ambiante et
- on mesure par photométrie la zone tachée par rapport à des zones de référence non tachées des mêmes vêtements au moyen des paramètres colorimétriques dans l'espace de couleur CIE L*a*b*.
